# EUROPÄISCHE PATENTSCHRIFT

(11) **EP 2 464 617 B1**
(45) Veröffentlichungstag und Bekanntmachung des Hinweises auf die Patenterteilung: **08.01.2014**
(21) Anmeldenummer: 09782832.1
(22) Anmeldetag: 09.09.2009
(51) Int. Cl.: C07C 29/151, C07C 29/50, C07C 31/04, C25B 1/04, C25B 15/00

(54) **VERFAHREN UND ANLAGE ZUM BEREITSTELLEN EINES KOHLENWASSERSTOFF-BASIERTEN ENERGIETRÄGERS UNTER EINSATZ EINES ANTEILS VON REGENERATIV ERZEUGTEM METHANOL UND EINES ANTEILS VON METHANOL, DER MITTELS DIREKTOXIDATION ODER ÜBER PARTIELLE OXIDATION ODER ÜBER REFORMIERUNG ERZEUGT WIRD**
METHOD AND SYSTEM FOR PROVIDING A HYDROCARBON-BASED ENERGY SOURCE USING A PORTION OF RENEWABLY PRODUCED METHANOL AND A PORTION OF METHANOL THAT IS PRODUCED BY MEANS OF DIRECT OXIDATION, PARTIAL OXIDATION, OR REFORMING
PROCÉDÉ ET INSTALLATION DE PRODUCTION D'UNE RESSOURCE ÉNERGÉTIQUE À BASE D'HYDROCARBURE EN UTILISANT UNE FRACTION DE MÉTHANOL PRODUIT PAR RÉGÉNÉRATION ET UNE FRACTION DE MÉTHANOL QUI EST PRODUIT PAR OXYDATION DIRECTE OU PAR OXYDATION PARTIELLE OU PAR REFORMAGE

(30) Priorität: 13.08.2009 WO PCT/EP2009/060472; 13.08.2009 EP 09167848
(43) Veröffentlichungstag der Anmeldung: 20.06.2012
(73) Patentinhaber: Silicon Fire AG, 6045 Meggen (CH)
(72) Erfinder: MEYER-PITTROFF, Roland, 85354 Freising (DE)
(74) Vertreter: OK pat AG
(86) Internationale Anmeldenummer: PCT/EP2009/061707
(87) Internationale Veröffentlichungsnummer: WO 2011/018124

(56) Entgegenhaltungen:
- WO-A2-00/25380
- US-A1- 2002 033 332
- US-A1- 2008 245 660
- US-A1- 2008 283 411

## Beschreibung

Die vorliegende Anmeldung betrifft Verfahren und Anlagen zum Bereitstellen speicherbarer und transportabler kohlenstoff-basierter Energieträger unter Einsatz von regenerativ erzeugtem Methanol und unter Einsatz von Methanol, das mittels Direktoxidation oder über partielle Oxidation oder über Reformierung erzeugt wird.

Es werden die Prioritäten der folgenden Anmeldungen beansprucht:
- internationale Patentanmeldung PCT/EP2009/060472 mit Anmeldedatum 13. August 2009, und
- europäische Anmeldung EP 09 167 848.2 mit Anmeldedatum 13. August 2009.

Kohlenstoffdioxid CO₂ (meist Kohlendioxid genannt) ist eine chemische Verbindung aus Kohlenstoff und Sauerstoff. Kohlendioxid ist ein farb- und geruchloses Gas. Es ist mit einer geringen Konzentration ein natürlicher Bestandteil der Luft und entsteht in Lebewesen bei der Zellatmung, aber auch bei der Verbrennung von kohlenstoffhaltigen Substanzen bei ausreichender Anwesenheit von Sauerstoff. Seit Beginn der Industrialisierung steigt der CO₂-Anteil in der Atmosphäre deutlich an. Hauptursache hierfür sind die vom Menschen verursachten - sogenannten anthropogenen - CO₂-Emissionen. Das Kohlendioxid in der Atmosphäre absorbiert einen Teil der Wärmestrahlung. Diese Eigenschaft macht Kohlendioxid zu einem so genannten Treibhausgas (THG) und zu einem der Mitverursacher des globalen Treibhauseffekts.

Aus diesen und auch aus anderen Gründen wird zur Zeit in verschiedenste Richtungen geforscht und entwickelt, um einen Weg zu finden, um die anthropogenen CO₂-Emissionen zu reduzieren. Besonders im Zusammenhang mit der Energieerzeugung, die häufig durch das Verbrennen fossiler Energieträger, wie Kohle, Öl oder Gas, erfolgt, aber auch mit anderen Verbrennungsprozessen, zum Beispiel der Müllverbrennung, besteht ein großer Bedarf zur Reduktion der CO₂-Emission. Es werden pro Jahr über 20 Milliarden Tonnen CO₂ durch solche Prozesse in die Atmosphäre abgegeben.

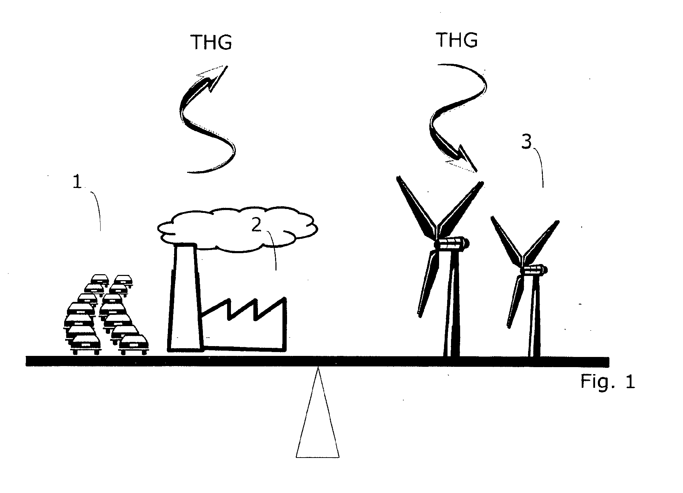

Es wird unter anderem das Prinzip der Klimaneutralität angestrebt, indem Ansätze verfolgt werden, bei denen man versucht, die mit CO₂-Emissionen verbundene Energieerzeugung durch alternative Energien zu kompensieren. Dieser Ansatz ist stark schematisiert in Fig. 1 dargestellt. Emittenten von Treibhausgasen (THG), wie Industrieunternehmen (z.B. Automobilhersteller) 1 oder Kraftwerksbetreiber 2, investieren oder betreiben z.B. Windfarmen 3 an anderen Standorten im Rahmen von Ausgleichsprojekten, um dort Energie ohne THG- Emissionen zu erzeugen. Rein rechnerisch kann sich damit eine Klimaneutralität ergeben. Zahlreiche Firmen versuchen, sich auf diesem Weg eine "klimaneutrale Weste" zu erkaufen.

Wind- und Solarkraftwerke, die erneuerbare Energien in elektrische Energie umwandeln, haben eine instationäre Leistungsabgabe, was einen Anlagenbetrieb gemäß den Anforderungen eines elektrischen Verbundnetzes außerordentlich erschwert und Anlage- und Betriebskosten für zusätzliche Reserve- und Frequenzregel-Anlagen bedingt. Die Stromerzeugungskosten von Wind- oder Solarkraftwerken werden dadurch gegenüber Kraftwerken, die den Leistungsanforderungen des Verbundnetzes unmittelbar folgen können, entsprechend zusätzlich wesentlich belastet.

Es wird als Problem angesehen, dass z.Z. nahezu alle regenerative elektrische Energie, die erzeugt wird, in das öffentliche Wechselspannungs-Verbundnetz eingespeist wird, dessen Frequenz nur innerhalb sehr enger Grenzen schwanken darf (z.B. +/- 0,4 %). Das kann nur erreicht werden, wenn die Stromerzeugung im Netz praktisch immer gleich dem Verbrauch ist. Die Notwendigkeit, für Wind- und Solarkraftwerke immer die ausreichenden Reserve- und Frequenzregelkapazitäten vorhalten zu müssen, führt zur entsprechenden Verteuerung der Stromversorgung mit diesen Anlagen. Wind- und Solarkraftwerke im elektrischen Verbundnetz ziehen somit weitere "versteckte" Kosten und Probleme nach sich.

Bereits beim heutigen Ausbaustand von Windkraftwerken in vielen Ländern kann das elektrische Energieversorgungsnetz vor ernste Probleme gestellt werden, wenn z.B. infolge von Windmangel oder Starkwind die Windleistung in großem Umfang ausfällt, vor allem wenn dieser Ausfall plötzlich und unerwartet erfolgt. In jedem Fall sind aber der installierten Wind- und Solarleistung angepasste Reserve- und Frequenzregelkapazitäten notwendig.

Daraus folgt, dass Solar- und Windkraftwerke, die in ein elektrisches Verbundnetz einspeisen, kaum die installierten Leistungen anderer Kraftwerke im Verbundnetz ersetzen können. Das führt dazu, dass Solar- und Windstrom in etwa nur mit den ersparten Brennstoffkosten der anderen, im Netz vorhandenen Wärmekraftwerke bewertet werden kann.

Aus dem Dokument US 2008/0283411 A1 sind ein Verfahren und eine Vorrichtung bekannt, die dazu dienen Kohlenwasserstoffe aus Kohlenstoff und Wasserstoff herzustellen. Gemäss diesem Dokument geht es um eine elektrolytische Produktion von Kohlenwasserstoffen. Es können unter anderem CO₂ und Wasser eingesetzt werden, um in einem elektrolytischen einen Alkohol, z.B. Methanol, zu erzeugen.

Aus dem Dokument US 2002/0033332 A1 ist ein System zur Elektrolyse von Wasser bekannt. Es kommt ein photovoltaisches System zum Einsatz, um als Stromquelle für die Elektrolyse von Wasser zu dienen. D.h. es kommt hier erneuerbare Energie zum Einsatz.

Das Dokument US 2008/0245660 A1 befasst sich mit dem Einsatz erneuerbarer Energie bei der Wasserstoff-Produktion und beim Einfangen von CO₂. Das CO₂ wird mit einem basischen Stoff zur Reaktion gebracht, der bei der Elektrolyse von Wasser entsteht. Insbesondere geht es um ein integriertes System.

Das Dokument WO 00/25380 betrifft die elektrische Energiespeicherung. Es geht konkret um eine Form der Energiespeicherung, bei der Wasser mit Strom aus einer Photovoltaikanlage zu Wasserstoff reduziert wird. Wasserstoff wird dann zusammen mit CO₂ zu Methanol umgesetzt. Das Methanol kann gespeichert werden. Wenn Bedarf für elektrische Energie besteht, wird das Methanol in Wasserstoff umgewandelt, um einen Verbrennungsmotor anzutreiben, oder um direkt oder indirekt Strom zu erzeugen.

Gemäß der eingangs erwähnten Prioritätsanmeldungen, lassen sich die regenerativen Energieformen besonders vorteilhaft mit fossilen Energieformen kombinieren. Eine solche Kombination ermöglicht es, kohlenwasserstoff-basierte Energieträger in entsprechenden Silicon-Fire Anlagen herzustellen. Diese Silicon-Fire Anlagen sind besonders geeignet, um damit Methanol herzustellen.

Es stellt sich nun die Aufgabe, ein Verfahren bereit zu stellen, das in der Lage ist, speicherbare kohlenwasserstoff-basierte Energieträger, zum Beispiel als Kraft- oder Brennstoffe, so zu erzeugen, dass die CO₂-(Gesamt-)Bilanz dieser Kraft- oder Brennstoffe gegenüber vorbekannten Ansätzen verbessert wird. Die Bereitstellung dieser Energieträger sollte mit möglichst minimalem Ausstoß von CO₂ erfolgen, und der Einsatz dieser Energieträger sollte zu einer Reduzierung des weltweiten Ausstoßes von CO₂ beitragen.

Gemäß Erfindung werden ein Verfahren und eine Anlage (Vorrichtung) zum Bereitstellen speicherbarer und transportabler Energieträger bereit gestellt.

Gemäß einer ersten Ausführungsform der Erfindung wird Kohlendioxid als Kohlenstofflieferant eingesetzt. Vorzugsweise wird das Kohlendioxid aus einem Verbrennungsprozess oder einem Oxidationsprozess von Kohlenstoff oder Kohlenwasserstoffen mittels CO₂-Abscheidung entnommen. Außerdem wird elektrische Gleichstromenergie bereitgestellt. Die Gleichstromenergie wird weitgehend mittels erneuerbarer Energien erzeugt, und sie wird zum Durchführen einer Elektrolyse von Wasser oder einer wässerigen Lösung eingesetzt, um Wasserstoff und Sauerstoff als Zwischenprodukte zu erzeugen. Das Kohlendioxid wird dann mit dem Wasserstoff zur Reaktion gebracht, um diese Produkte zu einem ersten Methanolanteil umzusetzen.

Ein zweiter Methanolanteil wird entweder durch Direktoxidation (oxidative Transformation) eines Kohlenwasserstoffes, vorzugsweise von methanhaltigem Erdgas, oder aus einem Synthesegas erzeugt, das im wesentlichen aus Kohlenmonoxid CO und Wasserstoff H₂ besteht. Dieses Synthesegas wird erzeugt entweder durch partielle Oxidation von Kohlenwasserstoffen, z.B. methanhaltigem Erdgas, mit Sauerstoff oder durch Reformieren von Kohlenstoff, z.B. Kohle, oder von Kohlenwasserstoffen, z.B. Erdgas, Ölen oder Biomassen, mit Sauerstoff und/oder Kohlendioxid und/oder Wasserdampf, wobei erfindungsgemäß der Sauerstoff für die Direktoxidation oder für die Herstellung des Synthesegases im Wesentlichen oder vollständig aus der gleichen Elektrolyse von Wasser oder einer wässerigen Lösung stammt, mit der auch der Wasserstoff für den ersten Methanolanteil hergestellt wird. Bei Bedarf kann dieser Sauerstoff
zwischengespeichert werden. Die Reformierung kann vorzugsweise auch als autotherme Reformierung durchgeführt werden, bei der die exotherme partielle

Oxidation und die endotherme Reformierung mit Wasserdampf und/oder Kohlendioxid so kombiniert werden, dass die kombinierte Gesamtreaktion ohne Wärmeaustausch mit der Umgebung, d.h. autotherm, verläuft.

Kohlendioxid als Kohlenstofflieferant kann gemäß Erfindung auch aus Roherdgas entnommen werden, das je nach Erdgasquelle über 10 % Kohlendioxid-Anteil aufweisen kann. Kohlendioxid kann z.B. auch aus Prozessen des Kalkbrennens oder des Kalzinierens zu Soda entstammen.

Gemäß Erfindung wird ein möglichst konstanter und langzeitiger Anlagenbetrieb einer entsprechenden Silicon-Fire Anlage angestrebt, was durch Kombination der Erzeugung des ersten (regenerativen) Methanolanteils mit der Erzeugung des zweiten (vorwiegend fossilen) Methanolanteils erzielt wird.

Die erfindungsgemäße Silicon-Fire Anlage wird so gesteuert und die einzelnen Prozesse werden so miteinander "verknüpft", dass
- der Gesamtertrag der Reaktionsprodukte möglichst maximal wird
- und/oder die CO₂-(Gesamt-)Emission möglichst minimal wird
- und/oder eine möglichst konstante und langzeitige Anlagenauslastung erzielt wird
- und/oder die produktspezifischen Investitions- und Betriebskosten des regenerativen Kraftwerkes und der Silicon-Fire Anlage möglichst minimal werden.

Die elektrische Energie aus Wind- und/oder Solarkraftwerken wird gemäß Erfindung nicht in einem Verbundnetz verbraucht, sondern in einer Silicon-Fire Anlage in Methanol als relativ einfach speicher- und transportierbare Energieform umgewandelt. D.h., es werden die erneuerbaren Energien auf chemischem Wege in eine relativ einfach speicher- und transportierbare Energieform überführt.

Es ist ein weiterer Vorteil des direkten Überführens in eine relativ einfach speicher- und transportierbare Energieform, dass die Energieumwandlungseffizienz erhöht wird, da bei Photovoltaikanlagen keine Wechselrichter zum Erzeugen von Wechselspannung erforderlich sind und generell ein verlustbehafteter Ferntransport der elektrischen Energie über längere Hochspannungsleitungen nicht erforderlich ist.

Außerdem kann der fossile gasförmige Rohstoff Methan in einem effizienten chemischen Prozess direkt zu flüssigem Methanol umgesetzt werden, das gegenüber einem Gas wiederum relativ einfacher speicher- und transportierbar ist.

Die Erzeugungskosten der erneuerbaren elektrischen Energie aus Solar- und Windkraftwerken sind auf absehbare Zeit relativ hoch. Das führt dazu, dass auch bei direkter Nutzung dieser elektrischen Energie für chemische Prozesse, wie hier vorgeschlagen, die allein damit erzeugten chemischen Produkte gegenüber den konventionell - in der Regel fossil - hergestellten Produkten wesentlich teurer sind.

Um diesen Nachteil zu vermindern, wird bei der erfindungsgemäßen Silicon-Fire Anlage vorzugsweise eine wirtschaftlich und ökologisch möglichst optimale Kombination der Herstellung eines ersten, weitgehend regenerativen Methanolanteils mit der Herstellung eines zweiten, vorzugsweise weitgehend fossilen Methanolanteils realisiert, wobei der für die Herstellung des zweiten Methanolanteils notwendige Sauerstoff weitgehend oder vollständig aus dem Elektrolyseprozess stammt, mit dem der Wasserstoff für den ersten Methanolanteil hergestellt wird.

In Zeiten elektrischen Spitzenbedarfs im elektrischen Verbundnetz kann die regenerative Energie - zur Erzielung höherer Erlöse - auch in das Verbundnetz eingespeist werden. Diese Einspeisung ist optional.

Vorzugsweise wird, anstatt die instationär anfallende regenerative elektrische Energie aus Wind- und/oder Solarkraftwerken in ein elektrisches Verbundnetz einzuspeisen und deren Schwankungen und Ausfälle durch andere Kraftwerke oder Speicheranlagen auszugleichen und auszuregeln, mit der elektrischen Energie der Wind- oder Solarkraftwerke eine chemische Silicon-Fire Anlage betrieben, um Methanol als relativ einfach speicher- und transportierbare Energieform zu erzeugen.

Die Produktion von Methanol als relativ einfach speicher- und transportierbare Energieform kann jederzeit heruntergefahren oder gar unterbrochen werden. Die "chemischen Anlagenteile" zur Herstellung sowohl des ersten, weitgehend regenerativen als auch des zweiten, vorzugsweise weitgehend fossilen Methanolanteils können relativ einfach und schnell heruntergefahren oder abgeschaltet werden. Hier liegt die Entscheidungshoheit im Verantwortungsbereich des Betreibers der Silicon-Fire Anlage.

Methanol kann als zusätzlicher Energiepuffer dienen. So kann zum Beispiel Methanol gespeichert werden, um bei Spitzen-Energiebedarf im elektrischen Verbundnetz zusätzlich elektrische Energie zur Verfügung stellen zu können. Methanol kann bei Bedarf entweder in Wärmekraftwerken verbrannt werden, oder es kann damit in Brennstoffzellen (z.B. Direkt-Methanol Brennstoffzellen; MFC genannt) elektrische Energie erzeugt werden.

Bei Bedarf kann das Methanol vor der Verbrennung katalytisch in ein Spaltgas aus Wasserstoff und Kohlenmonoxid überführt werden. Daraus ergeben sich Vorteile, die der detaillierten Beschreibung zu entnehmen sind.

Die vorliegende Erfindung basiert auf der Wasserstofferzeugung mit Hilfe elektrischer Energie, die weitmöglich regenerativ erzeugt wird und z.B. aus Wind- und/oder Solarkraftwerken stammt, in Kombination mit der Umsetzung des Wasserstoffs mit Kohlendioxid zu einem ersten Methanolanteil. Wasserstoff braucht also nicht gelagert oder hoch verdichtet oder tief gekühlt verflüssigt und über größere Strecken transportiert zu werden, sondern dient als Zwischenprodukt, das vorzugsweise am Standort seiner Erzeugung umgesetzt wird. Einem energieumwandelnden Prozess, bei dem Sonnenenergie oder Windkraft in elektrische Energie umgewandelt wird, folgen gemäß Erfindung stoffumwandelnde (chemische) Prozesse, nämlich die intermediäre Bereitstellung von Wasserstoff und die Umwandlung des Wasserstoffs zusammen mit Kohlendioxid zu dem ersten Methanolanteil. Der zweite Methanolanteil wird gemäß Erfindung auch durch stoffumwandelnde (chemische) Prozesse erzeugt, und zwar durch eine Direktoxidation von Kohlenwasserstoffen und/oder über eine partielle Oxidation von Kohlenwasserstoffen und/oder über eine Reformierung von Kohlenstoff oder Kohlenwasserstoffen, wobei der dafür notwendige Sauerstoff im Wesentlichen oder vollständig aus der Elektrolyse für den ersten Methanolanteil stammt.

Unter Beachtung entsprechender energietechnischer, anlagentechnischer und wirtschaftlicher Vorgaben, zusammen mit der Forderung nach schonender Nutzung aller stofflichen, energetischen und ökonomischen Ressourcen, wird gemäß Erfindung eine neue energietechnische Lösung bereitgestellt.

Weitere vorteilhafte Ausführungsformen sind der Beschreibung, den Figuren und den abhängigen Ansprüchen zu entnehmen.

In den Zeichnungen sind verschiedene Aspekte der Erfndung schematisch dargestellt, wobei die Zeichnungen zeigen:
- Fig. 1:: ein Schema, welches das Prinzip der Klimaneutralität durch die Investition in oder das Betreiben von Ausgleichsprojekten darstellt;
- Fig. 2:: ein Schema, das die grundlegenden Schritte des Verfahrens gemäß der eingangs erwähnten internationalen Prioritätsanmeldung, respektive einer entsprechenden Silicon-Fire Anlage, zeigt;
- Fig. 3:: ein Schema, das die grundlegenden Schritte des Verfahrens gemäß Erfindung, respektive einer entsprechenden Silicon-Fire Anlage, zeigt.

Das erfindungsgemäße Verfahren basiert auf einem neuartigen Konzept, welches unter Verwendung vorhandener Ausgangsstoffe sogenannte Reaktionsprodukte bereitstellt, die entweder direkt als Energieträger einsetzbar sind, oder die indirekt, d.h. nach dem Ausführen weiterer Zwischenschritte, als Energieträger einsetzbar sind.

Der Begriff Energieträger wird hier verwendet für Stoffe, die entweder direkt als Kraftstoff oder Brennstoff eingesetzt werden können. Hier geht es konkret um Methanol 108.1, 108.2.

Die Transportfähigkeit des Energieträgers wird hier durch das chemische Reaktionspotenzial gekennzeichnet.

Im Falle von Methanol 108.1, 108.2 als Energieträger sollten gewisse Rahmenbedingungen beim Lagern und beim Transport eingehalten werden, die ähnlich sind wie die Bedingungen zur Handhabung von anderen fossilen flüssigen Kraft- und Brennstoffen. Hier kann problemlos die existierende Infrastruktur benutzt werden. Auf der Materialseite können gewisse Anpassungen erforderlich sein, um z.B. den korrosiven Eigenschaften des Methanols Rechnung zu tragen. Auch die Sicherheitsmaßnahmen z.B. hinsichtlich Gesundheits-, Brand- und Explosionsschutz sind anzupassen.

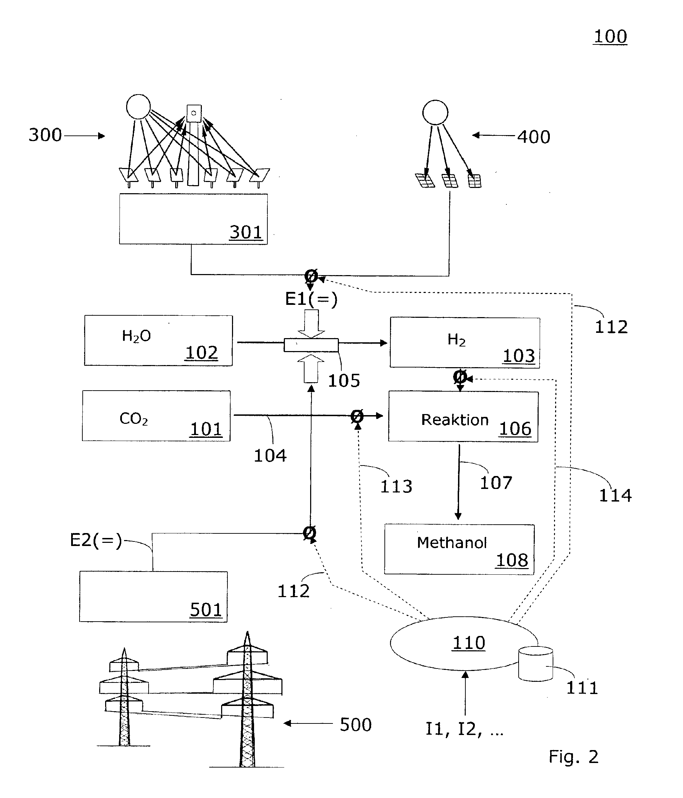

Fig. 2 zeigt in einer schematischen Blockdarstellung die wichtigsten Bausteine/Komponenten respektive Verfahrensschritte einer Silicon-Fire Anlage 100 gemäß der eingangs erwähnten internationalen Prioritätsanmeldung. Diese Anlage 100 ist so ausgelegt, dass ein Verfahren zum Bereitstellen speicherbarer und transportabler Energieträger 108 ausgeführt werden kann. Das entsprechende Verfahren basiert auf den folgenden grundlegenden Schritten.

Es wird Kohlenstoffdioxid 101 als Kohlenstofflieferant bereitgestellt. Die erforderliche elektrische Gleichstromenergie E1 wird hier weitmöglichst mittels erneuerbarer Energietechnik erzeugt und der Silicon-Fire Anlage 100 zur Verfügung gestellt. Besonders als erneuerbare Energietechnik geeignet sind Solarthermieanlagen 300 und Photovoltaikanlagen 400, die auf Solarmodulen basieren. Es ist auch möglich eine Kombination von beiden Anlagentypen 300 und 400 vorzusehen, da der Flächenbedarf, bezogen auf die elektrische Leistung, einer Solarthermieanlage 300 kleiner ist als der einer Photovoltaikanlage 400.

Es wird gemäß Fig. 2 eine Wasserelektrolyse 105 unter Einsatz der elektrischen Gleichstromenergie E1 durchgeführt, um Wasserstoff 103, oder Wasserstoffionen, als Zwischenprodukt zu erzeugen.

In Fig. 2 ist eine Anlage 100 dargestellt, die so aufgebaut ist, dass sie die eingangs erwähnten Nachteile vermindert oder ausgleicht. Aus diesem Grund wird bei der erfindungsgemässen Silicon-Fire Anlage 100 vorzugsweise eine wirtschaftlich und ökologisch optimale Kombination regenerativer Stromversorgung (durch die Anlagen 300 und/oder 400) und konventioneller Stromversorgung, hier durch einen Teil eines Verbundnetzes 500 dargestellt, realisiert. Diese Silicon-Fire Anlage 100 sieht daher vor, die regenerative elektrische Energie E1 weitgehend direkt entsprechend ihrem Anfall für chemische Reaktionen (hier die Elektrolysereaktion 105) zu nutzen und damit chemisch zu binden und zu speichern. Ein weiterer Anteil der benötigten Energie wird aus dem Verbundnetz 500 bezogen. Dieser Anteil wird umgewandelt in Gleichstrom(energie) E2. Zu diesem Zweck kommt ein entsprechender Umwandler 501 zum Einsatz, wie in Fig. 2 in schematischer Form angedeutet. Die entsprechenden Anlagenteile oder -komponenten werden hier auch als Energieversorgungsanlage 501 bezeichnet.

Mittels einer intelligenten Anlagensteuerung 110 wird die Energieversorgung der Anlage 100 nach Fig. 2 gesteuert und geregelt. Es wird im Prinzip der jeweils momentan verfügbare überschüssige Energieanteil E2 aus dem Verbundnetz 500 bezogen, während der andere Energieanteil (hier E1) soweit möglich aus einem Anlage bezogenen Solarkraftwerk 300 und/oder 400 (oder aus einem Windkraftwerk bezogen wird. Hier kommt es also zu einer intelligenten Umkehrung des bisherigen Prinzips, bei dem die Energieschwankungen erneuerbarer Energieanlagen 300, 400 durch Zu- und Abschalten konventioneller Anlagen abgefangen werden. Man braucht daher zum Betreiben einer Silicon-Fire Anlage 100 keine zusätzlichen Leistungs- und Frequenzregelkapazitäten für die regenerativen Kraftwerksanlagen im Verbundnetz 500 vorzuhalten. Dieses Prinzip ermöglicht es dem Betreiber einer Silicon-Fire Anlage 100, zusätzliche technische und wirtschaftliche Parameter bei der Steuerung der Anlage 100 einzubeziehen. Bei diesen Parametern handelt es sich um sogenannte Input-Größen I1, I2, usw., die von der Steuerung 110 in Entscheidungen einbezogen werden. Ein Teil der Parameter kann innerhalb der Steuerung 110 in einem Parameterspeicher 111 vorgegeben werden. Ein anderer Teil der Parameter kann von außen kommen. Hier können zum Beispiel Preis- und/oder Verfügbarkeitsinformationen vom Betreiber des Verbundnetzes 500 eingehen.

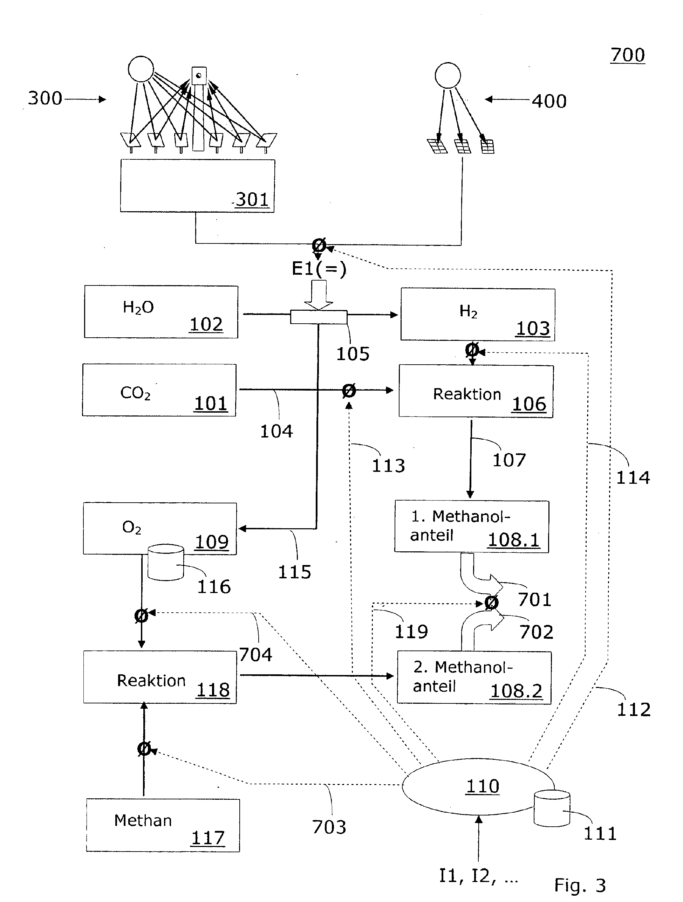

In Fig. 3 ist nun eine erfindungsgemäße Anlage 700 dargestellt, die so aufgebaut ist, dass sie die eingangs erwähnten Nachteile vermindert oder ausgleicht. Ein Teil dieser Anlage 700 entspricht der Anlage 100 nach Fig. 2. In dieser Beziehung wird daher auf die vorausgehende Beschreibung der entsprechenden Elemente verwiesen.

Es wird, wie beschrieben, durch eine Wasserelektrolyse 105 Wasserstoff 103 erzeugt, der umgesetzt wird zu einem ersten Methanolanteil 108.1. Die Energie hierzu stammt ganz oder weitestgehend (vorzugsweise zu mehr als 80%) aus regenerativen Energiequellen 300 und/oder 400. Bei der Wasserelektrolyse 105 entsteht als "Nebenprodukt" Sauerstoff 109. Dieser Sauerstoff 109, oder ein entsprechendes stark sauerstoffhaltiges Gas, kann über eine Sauerstoffentnahme 115 aus der Elektrolyseanlage 105 entnommen werden. In Fig. 3 ist ein optionaler Sauerstoffzwischenspeicher 116 gezeigt. Es wird nun in der gezeigten Ausführungsform Methan oder methanhaltiges Gas 117 bereitgestellt. Hier kann auch ein anderes kohlenwasserstoffhaltiges Gas zum Einsatz kommen. Dieses Methan oder methanhaltige Gas 117 kann aus einer Gasförderanlage oder zum Beispiel aus einer Biogasanlage stammen. Methan, respektive das methanhaltige Gas 117 wird nun in einer Reaktionsanlage 118 (z.B. mittels Direktoxidation) zu einem zweiten Methanolanteil 108.2 umgesetzt. Der erste Methanolanteil 108.1 und der zweite Methanolanteil 108.2 können zusammengebracht (gemischt) werden. Dieser optionale Schritt ist in Fig. 3 durch die Bezugszeichen 701 und 702 dargestellt. Die Steuerung 110 kann diesen Vorgang regeln und die Dosierung der Anteile 108.1 und 108.2 einstellen, wie durch die optionale Steuer- oder Signalleitung 119 dargestellt.

Es können einer Reihe weitere Steuer- oder Signalleitung vorgesehen sein, wie anhand der beispielhaft gezeigten Leitungen 112, 113 und 114 dargestellt. Diese Leitungen 112, 113 und 114 steuern Energie- oder Massenströme. Um die Massenströme auf der Eingangsseite der Reaktionsanlage 118 regeln zu können, können optionale Steuer- oder Signalleitung 703 und 704 vorgesehen sein.

In der Anlagensteuerung 110 sind sogenannte software-basierte Entscheidungsprozesse implementiert. Ein Prozessor der Steuerung 110 führt eine Steuerungssoftware aus und fällt unter Berücksichtigung von Parametern Entscheidungen. Diese Entscheidungen werden umgesetzt in Schalt- oder Steuerungsbefehle, die zum Beispiel über Steuer- oder Signalleitungen 112, 113, 114, 119, 703, 702 die Steuerung/Regelung von Energie- und Massenströmen bewirken.

Gemäß einer Ausführungsform der Erfindung wird Kohlendioxid 101 als Kohlenstofflieferant eingesetzt, wie in Fig. 3 schematisch angedeutet. Vorzugsweise wird das Kohlendioxid 101 aus einem Verbrennungsprozess 201 oder einem Oxidationsprozess über CO₂-Abscheidung (z.B. eine Silicon-Fire Rauchgasreinigungsanlage) entnommen. Das Kohlendioxid 101 kann aber auch von Roherdgas abgetrennt und bereitgestellt werden. Weiterhin wird elektrische Gleichstromenergie E1 bereitgestellt. Die Gleichstromenergie E1 wird weitgehend regenerativ (z.B. durch eine der Anlagen 300 und/oder 400 in Fig. 3) erzeugt. Die Gleichstromenergie E1 wird zum Durchführen einer Wasserelektrolyse eingesetzt, um Wasserstoff 103 als Zwischenprodukt zu erzeugen. Die Elektrolyseanlage, respektive das Durchführen einer solchen Elektrolyse, ist in Fig. 3 durch das Bezugszeichen 105 gekennzeichnet. Das Kohlendioxid 101 wird dann mit dem Wasserstoff 103 zur Reaktion (z.B. durch eine Methanolsynthese) gebracht, um die (Zwischen-)Produkte 101, 103 zu Methanol 108.1 umzusetzen. Die Reaktion kann in einem Reaktionsbehälter 106 durchgeführt werden, und die Entnahme, respektive das Bereitstellen des Methanols, ist in Fig. 3 durch das Bezugszeichen 107 gekennzeichnet.

Im Folgenden werden weitere grundlegende Details dieses Verfahrens und der entsprechenden Silicon-Fire Anlage 700 beschrieben.

Um Wasserstoff 103 als Zwischenprodukt erzeugen zu können, eignet sich eine Wasser-Elektrolyse unter Einsatz von Gleichstrom E1. Der benötigte Wasserstoff 103 wird in einer Elektrolyseanlage 105 durch die Elektrolyse von Wasser H₂O hergestellt:

H₂O - 286,02 kJ = H₂ + 0,5 O₂. (Reaktion 1)

Die benötigte (elektrische) Energie E1 für diese Reaktion von 286,02 kJ/mol entspricht 143010 kJ pro kg H₂.

Die Synthese des Methanols 108.1 (CH₃OH) verläuft in der Silicon-Fire Anlage 700 nach der exothermen Reaktion zwischen Kohlendioxid 101 (CO₂) und Wasserstoff 103 (H₂), wie folgt:

CO₂ + 3 H₂ = CH₃OH + H₂O - 49,6 kJ (Methanol gasförmig). (Reaktion 2)

Die entstehende Reaktionswärme W1 von 49,6 kJ/mol = 1550 kJ pro kg Methanol = 0,43 kWh pro kg Methanol wird aus dem entsprechenden Synthesereaktor 106 abgeführt. Typische Synthesebedingungen im Synthesereaktor 106 sind ca. 50 bar und ca. 270 °C, so dass die Reaktionswärme W1 auch genutzt werden kann, z.B. für eine in der Nähe befindliche Meerwasserentsalzungsanlage oder Heizungsanlage.

Vorzugsweise wird die Methanolsynthese 106 unter Einsatz von Katalysatoren durchgeführt, um Reaktionstemperatur und -druck sowie Reaktionsdauer niedrig zu halten und um sicher zu stellen, dass als Reaktionsprodukt hochwertiges (reines) Methanol 108.1 entsteht.

In einer anderen bevorzugten Ausführungsform der Erfindung wird eine Methanolsynthese nach einem von Prof. George A. Olah propagierten Elektrolyseverfahren durchgeführt. Details hierzu können zum Beispiel dem Buch "Beyond Oil and Gas: The Methanol Economy", George A. Olah et al., Wiley-VCH, 1998, ISBN 0-471-14877-6, Kapitel 11, Seite 196 entnommen werden. Weitere Details sind auch der US-Patentanmeldung US 2009/0014336 A1 zu entnehmen. Prof. George A. Olah beschreibt die Methanolsynthese durch Elektrolyse von CO₂ und H₂O wie folgt:

CO₂ + 2 H₂O - 682,01 kJ = CH₃OH + 1,5 O₂ (Reaktion 3)

Bei dieser Reaktion entstehen in einem Zwischenschritt CO und H₂ in einem Verhältnis von ungefähr 1:2. Außerdem entsteht O₂ 109, das in der Reaktionsanlage 118 weiterverwendet wird. Das CO und H₂, das an einer Kathode entsteht, kann mit einem kupfer- oder nickelbasierten Katalysator zu Methanol umgesetzt werden. Der Syntheseweg nach Reaktion 3 ist mit einer theoretischen Zufuhr von 682,01 kJ = 0,189 kWh elektrischer Energie pro Mol erzeugtem Methanol 108.1 verbunden.

Falls sich die Silicon-Fire Anlage 700 in der Nähe einer CO₂-Quelle befindet, kann auf eine Verflüssigung von CO₂ für den Transport verzichtet werden. Ansonsten ist es nach dem Stand der Technik relativ einfach, das CO₂ zu verflüssigen und auch über große Entfernungen zu einer Silicon-Fire Anlage 700 zu bringen. Bei einem Verzicht auf Verflüssigung, ggf. Speicherung und Transport über längere Entfernung ist das CO₂ unter Berücksichtigung von CO₂-Vermeidungs-Gutschriften voraussichtlich kostenneutral verfügbar. Auch im Falle eines Transports sind die Kosten für das "Erwerben" des CO₂ relativ gering.

Falls sich die Reaktionsanlage 118 der Silicon-Fire Anlage 700 in der Nähe der O₂-Quelle (hier z.B. der Elektrolyseur 105) befindet, kann auf eine Verflüssigung für den Transport und auf einen weiträumigen Transport selbst von O₂ 109 verzichtet werden.

In Fig. 3 ist anhand vom gestrichelten Pfeil 112 angedeutet, der von der Steuerung 110 ausgeht, dass die Steuerung 110 den Energiestrom E1 regelt. Der Pfeil 112 stellt eine Steuer- oder Signalleitung dar. Es sind auch andere mögliche Steuer- oder Signalleitungen 113, 114, 703, 704 dargestellt. Die Steuer- oder Signalleitung 113 regelt zum Beispiel die CO₂-Menge, die für die Reaktion 106 zur Verfügung steht. Wenn zum Beispiel weniger Wasserstoff 103 produziert wird, dann muss auch nur weniger CO₂ zugeführt werden. Die optionale Steuer- oder Signalleitung 114 kann zum Beispiel die H₂-Menge regeln. Eine solche Regelung ist zum Beispiel dann sinnvoll, wenn es einen Wasserstoff-Pufferspeicher gibt, dem man Wasserstoff 103 entnehmen kann, auch wenn im Moment kein Wasserstoff oder weniger Wasserstoff durch Elektrolyse 105 produziert wird. Die optionale Steuer- oder Signalleitung 703 kann die Zufuhr des Methans 117 und die optionale Steuer- oder Signalleitung 704 die Zufuhr des Sauerstoffs 109 regeln.

Untersuchungen haben ergeben, dass es besonders wirtschaftlich und umwelttechnisch sinnvoll ist, wenn die Silicon-Fire Anlage 100 so ausgelegt oder gesteuert wird, dass zwischen 15% und 40% als Methanolanteil 108.1 aus regenerativer Energie erzeugt wird, während die restlichen 85% bis 60% als Methanolanteil 108.2 aus Kohlenwasserstoffen (z.B. aus Methan 117) bereitgestellt werden.

Besonders bevorzugt ist eine Ausführungsform der Anlage 100, die den Bezug kostengünstiger elektrischer Energie in den Schwachlastzeiten aus einem Verbundnetz 500 vorsieht (wie in Fig. 2).

Die Aspekte der verschiedenen Ausführungsformen lassen sich problemlos durch eine entsprechende Auslegung der Steuerung 110 kombinieren.

Im Folgenden werden weitere grundlegende Aspekte eines erfindungsgemäßen Verfahrens zum Bereitstellen speicherbarer und transportabler Energieträger gezeigt.

Bekannt ist die Synthese von regenerativem Methanol CH₃OH aus Kohlendioxid CO₂ und Wasserstoff H₂, der über die (endotherme) Elektrolyse von Wasser mit regenerativer elektrischer Energie gemäß Reaktion 1, wie bereits weiter oben erwähnt, erzeugt wird.

H₂O - 286,02 kJ/mol = H₂ + 0,5O₂. (Reaktion 1)

Die exotherme Methanolsynthese (Reaktion 2, wie weiter oben bereits erwähnt) wird durch die Summenformel dargestellt:

CO₂ + 3 H₂ = CH₃OH + H₂O - 49,6 kJ (Methanol gasförmig). (Reaktion 2)

Die eingesetzte regenerative elektrische Energie E1 hat insbesondere im Fall von Wind- und Solarkraft die Nachteile, dass sie relativ teuer ist und nur unregelmäßig und mit zeitlichen Beschränkungen verfügbar ist.

Deshalb wird hier vorgeschlagen und beansprucht, die regenerative Herstellung von Methanol mit regenerativer elektrischer Energie E1 gemäß den Reaktionen 1 und 2 zu ergänzen durch eine Methanolsynthese (in der Reaktionsanlage 118) auf der Rohstoffbasis von Erdgas mit dem Hauptbestandteil Methan CH₄ 117, oder auf der Basis anderer Kohlenstoffausgangsmaterialien oder Kohlenwasserstoffausgangsmaterialien. Diese Synthese kann z.B. durch exotherme direkte Oxidation (oxidative Transformation, oxidative Konversion) des Methans 117 nach der Summenformel erfolgen

CH₄ + 0,5O₂ = CH₃OH - 167,5 kJ/mol. (Reaktion 4)

Diese Reaktion 4 wird hier als Direktoxidation bezeichnet.

Es kann aber auch der Weg über die partielle Oxidation (siehe Reaktion 5) oder die Reformierung (siehe Reaktionen 7 und/oder 8) (vorzugsweise als autotherme Reformierung) gewählt werden, um über den Weg von Synthesegas (hier im Wesentlichen Kohlenmonoxid und Wasserstoff umfassend) zu dem zweiten Methanolanteil 108.2 zu kommen.

Neu ist vor allem die Tatsache, dass der (reine) Sauerstoff 109 respektive das stark sauerstoffhaltige Gas, für Reaktion 4 oder auch für die partielle Oxidation oder die Reformierung direkt oder zwischengespeichert aus der Hydrolysereaktion 1 oder aus der Reaktion 3 stammt. Wird der (reine) Sauerstoff 109, respektive das stark sauerstoffhaltige Gas, aus der Hydrolysereaktion 1 genommen, so reicht gemäß den Massenverhältnissen der Reaktionen 1, 2, und 4 der Sauerstoff aus Reaktion 1 aus, um maximal drei Mal mehr Methanol 108.2 mit Reaktion 4 bereitzustellen als mit Reaktion 2, oder dass bei Verwendung allen Sauerstoffes 109 aus Reaktion 1 das gesamte erzeugte Methanol zu einem Viertel aus der "regenerativen" Reaktion 2 und zu drei Vierteln aus der "fossilen" Reaktion 4 (z.B. der Direktoxidation) stammt.

Durch Verringerung des Methanolanteils aus Reaktion 4 kann der "regenerative" Anteil 108.1 an der gesamten Methanolproduktion erhöht werden, z.B. auch mit entsprechenden Auswirkungen auf die CO₂-Bilanz der gesamten Methanolproduktion und die spezifischen CO₂-Emissionen bei der Verbrennung des "Gesamt"-Methanols zur Wärmeerzeugung oder als Kraftstoff.

In der Praxis wird vorzugsweise die zur Verfügung stehende regenerative elektrische Energie E1 zur Erzeugung von "regenerativem" Methanol 108.1 gemäß den Reaktionen 1 und 2 maximal ausgenutzt und der Anteil des nach Reaktion 4 erzeugten "fossilen" Methanols 108.2 wird nach wirtschaftlichen und ökologischen Zielsetzungen und Randbedingungen bis zum möglichen Maximalwert eingestellt, z.B. nach gewünschter spezifischer CO₂-Emission des "Gesamt"-Methanols bei Verbrennung oder nach dem momentanen Preis und der Verfügbarkeit des Erdgases oder nach der zu erzeugenden "Gesamt"-Methanolmenge oder nach den Preisen der verschiedenen Methanolanteile 108.1, 108.2.

Besonders vorteilhaft ist, dass das "regenerative" Methanols 108.1 und das "fossile" Methanol 108.2 in verschiedenen Reaktoren 106, 118 getrennt anfallen können und entweder getrennt abgegeben werden können oder nach dem Anfall und ggf. Zwischenlagerung in beliebigen Anteilen gemischt werden können (siehe Pfeile 701, 702), so dass die Silicon-Fire Anlage 700 rein "regeneratives" und rein "fossiles" Methanol liefern kann, aber auch beliebige Mischungen von beiden, um z.B. als regenerativer Kraftstoff mit zulässigem fossilen Anteil oder zulässiger spezifischer CO₂-Emission vermarktet werden zu können.

Das Herstellungsverhältnis ein Viertel "regeneratives" Methanol 108.1 und drei Viertel "fossiles" Methanol 108.2 entspricht dem rechnerischen solaren (Peak-)Energieanfall bezogen auf die Zeitdauer eines Kalenderjahres, d.h. die Silicon-Fire Anlage 700 könnte rechnerisch während ca. eines Viertels der Jahreszeitdauer "regeneratives" und während ca. drei Vierteln der Jahreszeitdauer "fossiles" Methanol produzieren, wenn der für das "fossile" Methanol benötigte Sauerstoff 109 entsprechend zwischengespeichert wird. Hierzu kann der Zwischenspeicher 116 dienen.

Die Reaktionswärmen der exothermen Reaktionen 2 und 4 werden vorzugsweise genutzt, wodurch sich auch die anzurechnenden spezifischen CO₂-Emissionen der verschiedenen Methanolfraktionen 108.1, 108.2 verringern würden. Für die Wärmenutzung kommt z.B. unter den Bedingungen des Persischen Golfes sehr vorteilhaft die Beheizung von thermischen Seewasserentsalzungs-Anlagen in Frage, insbesondere während der kälteren Jahreszeit, wenn wegen des wesentlich geringeren Strombedarfes für die Gebäudeklimatisierung Wärmekraftwerke in Teillast gefahren oder sogar abgeschaltet werden und deren Abwärme nicht mehr (ausreichend) für die ihnen angeschlossenen Seewasserentsalzungs-Anlagen zur Verfügung steht.

Die Nutzung des bei der Elektrolyse 105 entstehenden großen Sauerstoff-Massenstromes für Reaktion 4 oder für die partielle Oxidation oder die Reformierung vermeidet die ökologischen Risiken, die sonst bei Emission in die Umgebung auftreten können.

Die Reaktion 4 wird z.B. erwähnt in Olah, G. et al., "Behind Oil and Gas: The Methanol Economy", Wiley-VCH Verlag, 2006, S. 171 und 172 als "direct oxidative transformation of natural gas to methanol" oder als "producing methanol from natural gas".

Ein zweistufiger Oxidationsprozess, der bei 500 und 350 °C abläuft, wird beschrieben von Mantashyan, A. A. et al. in "Non isothermal oxidative conversion of methane to methanol ...", Hayastani Kimiakan Handes (2007), 60(5), S. 887-897. Weitere neuere Literaturquellen für die Reaktion 4 sind z.B.:

Holmen, A., "Direct conversation of methane to fuels and chemicals", Catalysis Today (2009), 142(1-2), S. 8;

Ankan, P. et al., "Mechanism and energetics of methane to methanol conversation", Organometallics (2007), 26(4), 793-809.

Das Ergebnis der Reaktion 4 kann aber z.B. auch durch die Kombination der folgenden großtechnisch erprobten Reaktionen 5 und 6 erreicht werden.

Die partielle Oxidation von Methan 117:

CH₄ + 0,5O₂ = CO + 2H₂ -36 kJ/mol (Reaktion 5)

und die klassische Methanolsynthese (z.B. 250 bar, 350 °C, mit Chromoxid-Zinkoxid-Katalysator):

CO + 2H₂ = CH₃OH -128,20 kJ/mol (flüss. Methanol). (Reaktion 6)

Bei der Kombination der Reaktionen 5 und 6 findet quasi auch eine Oxidation des methanhaltigen Gases 117 unter Einsatz von sauerstoffhaltigem Gas 109 statt.

Die Reaktion 6 kann unter Umständen im gleichen Reaktor ablaufen wie die Reaktion 2, evtl. mit Wechsel oder Anpassung der Katalysatorfüllung und der Katalysebedingungen.

Die Reformierung von Methan mit Wasserdampf (steam reforming) zu Synthesegas läuft endotherm nach der folgenden Reaktion ab:

CH₄ + H₂O (gasf.) = CO + 3H₂ + 206,2 kJ/mol (Reaktion 7)

Die Reformierung von Methan mit Kohlendioxid zu Synthesegas läuft endotherm nach der folgenden Reaktion ab:

CH₄ + CO₂ = 2CO + 2H₂ + 247 kJ/mol (Reaktion 8)

Die drei Reaktionen 5, 7 und 8 können gemeinsam in einem Reaktor bei Temperaturen von ca. 800 - 1000 °C über Katalysatoren ablaufen und so gesteuert werden, dass sie möglichst energieautark ("autotherm") ablaufen und die Reaktionsprodukte ein geeignetes Synthesegas für die klassische Methanolsynthese nach Reaktion 6 ergeben.

Weitere Details sind z.B. der Publikation von Bharadwaj, S.S.; L.D. Schmidt: Catalytic partial oxidation of natural gas to syngas. Fuel Processing Technology 42 (1995), S. 109 - 127 zu entnehmen.

Gemäß Erfindung dient CO₂ 101 als Ausgangsstoff und Kohlenstofflieferant für die Methanolsynthese im Reaktor 106. Als CO₂-Quellen dienen vorzugsweise: Steam-Reforming-Anlagen, CO₂-Abscheidungsanlagen für Roherdgas, Kalkbrennöfen, Kalzinieranlagen für Soda, Fermentationsanlagen für Bioethanol, Meerwasserentsalzungsanlagen, große Feuerungsanlagen für fossile Brennstoffe (z.B. Kraftwerksfeuerungen) und andere Anlagen oder Verbrennungsprozesse, die relativ große Mengen an CO₂ emittieren.

Die Erfindung ermöglicht es, die erheblichen wirtschaftliche Nachteile bekannter Ansätze zu vermeiden, wenn - wie im Fall der Silicon-Fire Anlage 700 - die instationär anfallende elektrische Solar- und/oder Windenergie direkt in chemische Reaktionsenthalpie umgewandelt und chemisch gebunden gespeichert wird, ohne dass zusätzliche Kapazitäten für Reserveleistungen und/oder Frequenzregelung im Verbundnetz und die dafür erforderlichen Aufwendungen notwendig sind.

Bei photovoltaischer Stromerzeugung mittels einer Photovoltaikanlage 400 ist ein weiterer Vorteil, dass der aus den Solarzellen der Photovoltaikanlage 400 primär anfallende Gleichstrom E1 direkt für den chemischen Prozess (Elektrolyse 105) genutzt werden kann, ohne über Umrichter in Wechselstrom zur Spannungstransformation umgewandelt werden zu müssen.

In einer anderen Ausführungsform der Erfindung kann auch ein ATR-Prozess (Autotherme Reformierung) für die Verarbeitung eines Ausgangsmaterials eingesetzt werden. Bei der autothermen Reformierung wird ein kohlenwasserstoffhaltiges oder ein kohlenstoffhaltiges Ausgangsmaterial in einer Reaktionszone in Gegenwart einer unterstöchiometrischen Menge an Sauerstoff (z.B. Sauerstoff 109) oxidiert, die für die vollständige Oxidation nicht ausreicht. Außerdem werden Wasserdampf und/oder Kohlendioxid zugeführt, um auf diesem Weg Synthesegas erzeugen zu können, das im Wesentlichen Kohlenmonoxid und Wasserstoff umfasst. Das Ausgangsmaterial kann Erdgas oder ein anderer Kohlenwasserstoff sein. Es ist auch möglich Kohlen, Öle, Brenngase, Biomassen, Ölsande oder Ölschiefer mit dem ATR-Prozess zu einem geeigneten Synthesegas umzusetzen.

In einer anderen Ausführungsform der Erfindung wird beim ATR-Prozess Kohlendioxid zugeführt. Das Hinzufügen von CO₂ kann dann von Vorteil sein, wenn die stöchiometrischen Verhältnisse aufgrund der Ausgangsmaterialien für die Synthese von Methanol nicht optimal sind.

Bei den Ausführungsformen, die auf einem ATR-Prozess beruhen, wird Synthesegas erzeugt, das im Wesentlichen Kohlenmonoxid und Wasserstoff umfasst. Aus diesem Synthesegas wird dann in einem nachgelagerten Schritt Methanol synthetisiert, wie z.B. in Reaktion 6 gezeigt.

In einer besonders bevorzugten Ausführungsform der Erfindung wird das Methanol 108.1, 108.2 als Energieträger zum Lagern und Transportieren eingesetzt.

Kohlendioxid als Kohlenstofflieferant kann gemäß Erfindung auch aus dem Roherdgas entnommen werden, das je nach Erdgasquelle mehr als 10 % Kohlenstoffdioxid-Anteil aufweisen kann. Nach dem Fördern des Roherdgases wird bereits heute typischerweise eine Gaswäsche (mittels Gaswäschetechnologie oder einer anderen Gastrenntechnologie) durchgeführt, um das CO₂ vom eigentlichen Erdgas zu trennen. Meist wird dieses CO₂ in die Atmosphäre emittiert. Gemäß Erfindung kann das CO₂, das in weitgehend reiner Form vorliegt, als Kohlenstofflieferant 101 eingesetzt werden.

Für die Nutzung des Methanols 108.1, 108.2 in Verbrennungskraftmaschinen, z.B. auch in kombinierten Gas-/Dampfturbinenanlagen, kann eine katalytische Spaltung (z.B. bei ca. 380 °C) vor der Verbrennung nach der Reaktion 9:

CH₃OH (flüssig) = 2H₂ + CO +128,20 kJ/mol (Reaktion 9)

folgende Vorteile bringen:

Eine normalerweise mit Gas betriebene Verbrennungskraftmaschine braucht nicht auf einen flüssigen Brennstoff umgestellt zu werden. Die Reaktionswärme der endothermen Methanol-Spaltreaktion 9 von 128,20 kJ/mol oder 4006 kJ/kg Methanol kann durch die Abgasabwärme einer Verbrennungskraftmaschine aufgebracht werden, wodurch sich der ursprüngliche Heizwert des Methanols von 19900 kJ/kg um die zugeführte Reaktionswärme um ca. 20 % erhöht. Die Anwendung einer entsprechenden Methanolspaltung für Kraftwerksprozesse wird z.B. beschrieben in Mußenbrock, K: "Möglichkeiten zur Nutzung von Methanol in Kraftwerksprozessen", VGB Kraftwerkstechnik 71 (1991), Heft 8, S. 759 - 764.

**Bezugszeichen:**

| | |
|---|---|
| Fahrzeugindustrie / Automobilbau | 1 |
| Kraftwerksbetreiber | 2 |
| Windfarm | 3 |
| Silicon-Fire Anlage (aus Parallelanmeldung) | 100 |
| Kohlenstoffdioxid | 101 |
| Wasser | 102 |
| Wasserstoff | 103 |
| Bereitstellen von Kohlenstoffdioxid | 104 |
| Durchführen einer Elektrolyse | 105 |
| Zusammenführen des Wasserstoffs (H₂) und des Kohlenstoffdioxids / Synthesereaktor | 106 |
| Abgeben/Bereitstellen von Methanol | 107 |
| transportabler Energieträger | 108 |
| Sauerstoff oder sauerstoffhaltiges Gas | 109 |
| (Anlagen-)Steuerung | 110 |
| Parameterspeicher | 111 |
| Steuer- oder Signalleitungen | 112, 113, 114 |
| Sauerstoffentnahme | 115 |
| Optionaler Sauerstoffzwischenspeicher | 116 |
| Methan oder methanhaltiges Gas | 117 |
| Reaktionsanlage | 118 |
| Steuer- oder Signalleitung | 119 |
| Solarthermieanlage | 300 |
| Umwandlung von Wärme in Gleichstrom | 301 |
| Solaranlage (Photovoltaikanlage) | 400 |
| Verbundnetz | 500 |
| Umwandlung von Wechselspannung in Gleichstrom | 501 |
| (Energieversorgungsanlage) | |
| siliziumdioxidhaltiges Ausgangsmaterial | 601 |
| Silizium | 603 |
| Reduktionsverfahren | 602 |
| Siliziumdioxid als Rückreaktionsprodukt | 604 |
| Hydrolyse | 605 |
| | |
| Silicon-Fire Anlage (Erfindung) | 700 |
| Pfeile | 701, 702 |
| Steuer- oder Signalleitungen | 703, 704 |
| Gleichstromenergie | E1 |
| Inputgrößen | I1, I2, usw. |
| Primärenergie | P1, P2 |

## Patentansprüche

1. Verfahren zum Bereitstellen speicherbarer und transportabler Energieträger (108) mit den folgenden Schritten:
- Bereitstellen (104) von Kohlenstoffdioxid (CO₂; 101) als Kohlenstofflieferant,
- Bereitstellen von elektrischer Gleichstromenergie (E1), die mittels erneuerbarer Energietechnik (300, 400) erzeugt wird,
- Durchführen einer Elektrolyse (105) unter Einsatz dieser elektrischen Gleichstromenergie (E1), um Wasserstoff (H₂; 103) als Zwischenprodukt und stark sauerstoffhaltiges Gas (109) zu erzeugen,
- Zusammenführen (106) des Wasserstoffs (H₂; 103) und des Kohlenstoffdioxids (CO₂; 101), um dieses umzusetzen zu einem ersten Methanolanteil (108.1),
- Bereitstellen des stark sauerstoffhaltigen Gases (109), das bei der Durchführung der Elektrolyse (105) ensteht,
- Bereitstellen von kohlenwasserstoffhaltigem Gas(117),
- Durchführen einer Oxidation (118) des kohlenwasserstoffhaltigen Gases (117) unter Einsatz des stark sauerstoffhaltigen Gases (109), um das kohlenwasserstoffhaltige Gas (117) umzusetzen zu einem zweiten Methanolanteil (108.2).

2. Verfahren nach Anspruch 1, **dadurch gekennzeichnet, dass** in einem weiteren Schritt (701, 702) der erste Methanolanteil (108.1) mit dem zweiten Methanolanteil (108.2) zusammengeführt wird, um den speicherbaren und transportablen Energieträger (108) zu erhalten.

3. Verfahren nach Anspruch 1 oder 2, **dadurch gekennzeichnet, dass** das stark sauerstoffhaltige Gas (109) beim Durchführen einer Direktoxidation zum Einsatz kommt, wobei durch diese Direktoxidation der zweite Methanolanteil (108.2) erzeugt wird.

4. Verfahren nach Anspruch 1, 2 oder 3, **dadurch gekennzeichnet, dass** das stark sauerstoffhaltige Gas (109) zwischengelagert wird.

5. Verfahren nach einem der vorhergehenden Ansprüche, **dadurch gekennzeichnet, dass** das Entnehmen von Kohlenstoffdioxid (CO₂; 101) aus einem Verbrennungsprozess oder einem Oxidationsprozess mittels CO₂-Abscheidung erfolgt.

6. Verfahren nach einem der vorhergehenden Ansprüche 1 bis 5, **dadurch gekennzeichnet, dass** das Kohlenstoffdioxid (CO₂; 101) aus einer Anlage entnommen wird, die Roherdgas beim Reinigen von einem Kohlenstoffdioxidanteil befreit.

7. Verfahren nach Anspruch 1 oder 2, **dadurch gekennzeichnet, dass** es sich bei der Elektrolyse um eine Wasserelektrolyse (105) handelt, bei der direkt aus Wasser oder einer wässerigen Lösung (H₂O; 102) Wasserstoff (H₂; 103) und das stark sauerstoffhaltige Gas (109) erzeugt werden.

8. Verfahren nach einem der vorhergehenden Ansprüche, **dadurch gekennzeichnet, dass** die Gleichstromenergie (E1) durch Sonnenlicht mittels einer Solarthermie-Anlage (400) und/oder einer Photovoltaikanlage (300) geliefert wird.

9. Verfahren nach einem der vorhergehenden Ansprüche, **dadurch gekennzeichnet, dass** es sich bei dem kohlenwasserstoffhaltigen Gas um methanhaltiges Gas handelt.

10. Verfahren nach einem der vorhergehenden Ansprüche 1 - 9, **dadurch gekennzeichnet, dass** das kohlenwasserstoffhaltige Gas aus kohlenstoffhaltigem Material hergestellt wird.

11. Verfahren nach einem der vorhergehenden Ansprüche 1 - 9, **dadurch gekennzeichnet, dass** mittels Reformierung über den Weg von Synthesegas der zweite Methanolanteil (108.2) erzeugt wird.

12. Verfahren nach Anspruch 11, **dadurch gekennzeichnet, dass** es sich bei der Reformierung um eine autotherme Reformierung handelt.

13. Verfahren nach einem der vorhergehenden Ansprüche, **dadurch gekennzeichnet, dass** zu einem späteren Zeitpunkt der erste Methanolanteil (108.1) und/oder der zweite Methanolanteil (108.2) umgesetzt wird zu Spaltgas, bestehend im Wesentlichen aus Kohlenmonoxid und Wasserstoff.

14. Verfahren nach einem der vorhergehenden Ansprüche, **dadurch gekennzeichnet, dass** in einem Zwischenschritt vor der Umsetzung zu dem zweiten Methanolanteil (108.2) ein Synthesegas erzeugt wird, das im Wesentlichen aus Kohlenmonoxid CO und Wasserstoff H₂ besteht, wobei dieses Synthesegas durch eine partielle Oxidation von Kohlenwasserstoffen erzeugt wird.

15. Anlage (700) zum Bereiststellen eines speicherbaren und transportablen Energieträgers (108.1, 108.2), mit
- einer Anlage (300, 301; 400) zum Erzeugen eines ersten Energieanteils in Form von Gleichstromenergie (E1) aus mindestens einer erneuerbaren Energiequelle,
- einer Elektrolysevorrichtung (102, 105) zum Bereitstellen von Wasserstoff (103) als Zwischenprodukt und zum Bereitstellen von stark sauerstoffhaltigem Gas (109), wobei der Energiebedarf dieser Elektrolysevorrichtung (102, 105) im Wesentlichen, d.h. zu mehr als 80%, durch den ersten Energieanteil (E1) gedeckt wird,
- einer Kohlendioxidzufuhr zum Einbringen von Kohlendioxid (101),
- einem Reaktionsbereich (106) zum Herstellen eines ersten Methanolanteils (108.1), wobei in dem Reaktionsbereich (106) der Wasserstoff (103) mit dem Kohlendioxid (101) zur Reaktion gelangt,
- einer Reaktionsanlage (118) zum Herstellen eines zweiten Methanolanteils (108.2), wobei in der Reaktionsanlage (118) das stark sauerstoffhaltige Gas (109) mit einem kohlenwasserstoffhaltigen Gas (117) zu einer Oxidationsreaktion gelangt,
- einer Entnahme (701) für den ersten Methanolanteil (108.1), und mit
- einer Entnahme (702) für den zweiten Methanolanteil (108.2).

16. Anlage (700) nach Anspruch 15, **dadurch gekennzeichnet, dass** sie Mittel zum Bereitstellen von Methanol in Form einer Mischung aus dem ersten Methanolanteil (108.1) und dem zweiten Methanolanteil (108.2) umfasst.

17. Anlage (700) nach Anspruch 15 oder 16, **dadurch gekennzeichnet, dass** sie eine Steuerung (110) umfasst, die dazu ausgelegt ist, die Verfahren, die in den einzelnen Anlageteilen ablaufen, zu steuern.

18. Anlage (700) nach Anspruch 17, **dadurch gekennzeichnet, dass** in der Steuerung (110) ein software-basierter Entscheidungsprozess implementiert ist, damit zwischen 15% und 40% als erster Methanolanteil (108.1) und die restlichen 85% bis 60% als zweiter Methanolanteil (108.2) bereitgestellt werden.

19. Anlage (700) nach einem der Ansprüche 15 bis 18, **dadurch gekennzeichnet, dass** mit der Steuerung (110) über Steuer- oder Signalleitungen (112, 113, 114, 703, 704) Energie- und/oder Massenströme in der Anlage (700) geregelt und/oder gesteuert werden.

20. Anlage (700) nach einem der Ansprüche 15 bis 19, **dadurch gekennzeichnet, dass** sie einen Reaktionsbereich zum Herstellen eines Synthesegases umfasst, das im Wesentlichen aus Kohlenmonoxid CO und Wasserstoff H₂ besteht, wobei dieses Synthesegas in dem Reaktionsbereich durch eine partielle Oxidation von Kohlenwasserstoffen erzeugt wird.

21. Anlage (700) nach einem der Ansprüche 15 bis 20, **dadurch gekennzeichnet, dass** die Reaktionsanlage (118) als Anlage zur Herstellung des zweiten Methanolanteils (108.2) über den Weg von Synthesegas aus Reformierungsreaktionen ausgelegt ist.

22. Anlage (700) nach Anspruch 21, **dadurch gekennzeichnet, dass** es sich bei der Reformierung um eine autotherme Reformierung handelt.

23. Anlage (700) nach einem der vorhergehenden Ansprüche 15 bis 22, **dadurch gekennzeichnet, dass** sie dazu ausgelegt ist, aus dem ersten Methanolanteil (108.1) und/oder dem zweiten Methanolanteil (108.2) Spaltgas, bestehend im Wesentlichen aus Kohlenmonoxid und Wasserstoff, zu erzeugen.

## Claims

1. A method for providing storable and transportable energy carriers (108) having the following steps:
- providing (104) carbon dioxide (CO₂; 101) as a carbon supplier,
- providing electrical direct-current energy (E1), which is generated by means of renewable energy technology (300, 400),
- carrying out an electrolysis (105) by employing this electrical direct-current energy (E1) in order to produce hydrogen (H₂; 103) as an intermediate product and strongly oxygenated gas (109),
- combining (106) the hydrogen (H₂; 103) and the carbon dioxide (CO₂; 101), in order to react them to form a first methanol fraction (108.1),
- providing the strongly oxygenated gas (109), which arises as the electrolysis (105) is carried out,
- providing gas (117) containing hydrocarbons,
- carrying out an oxidation (118) of the gas (117) containing hydrocarbons by using the strongly oxygenated gas (109), in order to react the gas (117) containing hydrocarbons to form a second methanol fraction (108.2).

2. The method according to Claim 1, **characterized in that**, in a further step (701, 702), the first methanol fraction (108.1) is combined with the second methanol fraction (108.2), to obtain the storable and transportable energy carrier (108).

3. The method according to Claim 1 or 2, **characterized in that** the strongly oxygenated gas (109) is used when carrying out direct oxidation, wherein the second methanol fraction (108.2) is produced by this direct oxidation.

4. The method according to Claim 1, 2, or 3, **characterized in that** the strongly oxygenated gas (109) is buffered.

5. The method according to one of the preceding claims, **characterized in that** the removal of carbon dioxide (CO₂; 101) from a combustion process or an oxidation process is performed by means of CO₂ precipitation.

6. The method according to one of preceding Claims 1 to 5, **characterized in that** the carbon dioxide (CO₂; 101) is withdrawn from a facility which frees a carbon dioxide fraction from crude natural gas during purification.

7. The method according to Claim 1 or 2, **characterized in that** the electrolysis is a water electrolysis (105), during which hydrogen (H₂; 103) and the strongly oxygenated gas (109) are produced directly from water or an aqueous solution (H₂O; 102).

8. The method according to one of the preceding claims, **characterized in that** the direct-current energy (E1) is supplied by sunlight by means of a solar thermal energy facility (400) and/or a photovoltaic facility (300).

9. The method according to one of the preceding claims, **characterized in that** the gas containing hydrocarbons is methane-containing gas.

10. The method according to one of preceding Claims 1 to 9, **characterized in that** the gas containing hydrocarbons is produced from carbonaceous material.

11. The method according to one of preceding Claims 1 to 9, **characterized in that** the second methanol fraction (108.2) is produced by means of reformation via the pathway of synthesis gas.

12. The method according to Claim 11, **characterized in that** the reformation is an autothermal reformation.

13. The method according to one of the preceding claims, **characterized in that**, at a later point in time, the first methanol fraction (108.1) and/or the second methanol fraction (108.2) are reacted to form cracked gas, substantially consisting of carbon monoxide and hydrogen.

14. The method according to one of the preceding claims, **characterized in that**, in an intermediate step before the reaction to form the second methanol fraction (108.2), a synthesis gas is produced, which substantially consists of carbon monoxide CO and hydrogen H₂, wherein this synthesis gas is produced by partial oxidation of hydrocarbons.

15. A facility (700) for providing a storable and transportable energy carrier (108.1, 108.2), having
- a facility (300, 301; 400) for producing a first energy fraction in the form of direct-current energy (E1) from at least one renewable energy source,
- an electrolysis device (102, 105) for providing hydrogen (103) as an intermediate product and for providing strongly oxygenated gas (109), wherein the energy consumption of this electrolysis device (102, 105) is essentially covered, i.e., more than 80%, by the first energy fraction (E1),
- a carbon dioxide supply for introducing carbon dioxide (101),
- a reaction region (106) for producing a first methanol fraction (108.1), wherein the hydrogen (103) reacts with the carbon dioxide (101) in the reaction region (106),
- a reaction facility (118) for producing a second methanol fraction (108.2), wherein the strongly oxygenated gas (109) reacts with a gas (117) containing hydrocarbons in an oxidation reaction in the reaction facility (118),
- a withdrawal (701) for the first methanol fraction (108.1), and having
- a withdrawal (702) for the second methanol fraction (108.2).

16. The facility (700) according to Claim 15, **characterized in that** it comprises means for providing methanol in the form of a mixture of the first methanol fraction (108.1) and the second methanol fraction (108.2).

17. The facility (700) according to Claim 15 or 16, **characterized in that** it comprises a controller (110), which is designed for the purpose of controlling the methods which run in the individual facility parts.

18. The facility (700) according to Claim 17, **characterized in that** a software-based decision process is implemented in the controller (110), so that between 15% and 40% is provided as the first methanol fraction (108.1) and the remaining 85% to 60% is provided as the second methanol fraction (108.2).

19. The facility (700) according to one of Claims 15 to 18, **characterized in that** energy and/or mass flows in the facility (700) are regulated and/or controlled using the controller (110) via control or signal lines (112, 113, 114, 703, 704).

20. The facility (700) according to one of Claims 15 to 19, **characterized in that** it comprises a reaction region for producing a synthesis gas, which substantially consists of carbon monoxide CO and hydrogen H₂, wherein this synthesis gas is produced in the reaction region by partial oxidation of hydrocarbons.

21. The facility (700) according to one of Claims 15 to 20, **characterized in that** the reaction facility (118) is designed as a facility for producing the second methanol fraction (108.2) via the pathway of synthesis gas from reformation reactions.

22. The facility (700) according to Claim 21, **characterized in that** the reformation is an autothermal reformation.

23. The facility (700) according to one of preceding Claims 15 to 22, **characterized in that** it is designed for the purpose of producing cracked gas, substantially consisting of carbon dioxide and hydrogen, from the first methanol fraction (108.1) and/or the second methanol fraction (108.2).

## Revendications

1. Procédé de production d'une ressource énergétique stockable et transportable (108) comprenant les étapes consistant à :
- produire (104) du dioxyde de carbone (CO₂ ; 101) comme source de carbone,
- produire une énergie électrique continue (E1) produite au moyen d'une technique énergétique renouvelable (300, 400),
- réaliser une électrolyse (105) en utilisant cette énergie électrique continue (E1) pour produire de l'hydrogène (H₂ ; 103) comme produit intermédiaire et du gaz riche en oxygène (109),
- assembler (106) l'hydrogène (H₂ ; 103) et le dioxyde de carbone (CO₂ ; 101) pour le transformer en une première fraction de méthanol (108.1),
- produire le gaz riche en oxygène (109) qui résulte de la réalisation de l'électrolyse (105),
- produire le gaz contenant les hydrocarbures (117),
- réaliser une oxydation (118) du gaz contenant des hydrocarbures (117) en utilisant le gaz riche en oxygène (109) pour transformer le gaz contenant les hydrocarbures (117) en une deuxième fraction de méthanol (108.2).

2. Procédé selon la revendication 1, **caractérisé en ce que**, dans une autre étape (701, 702), la première fraction de méthanol (108.1) est assemblée à la deuxième fraction de méthanol (108.2) pour obtenir la ressource énergétique stockable et transportable (108).

3. Procédé selon la revendication 1 ou 2, **caractérisé en ce que** le gaz riche en oxygène (109) est utilisé lorsqu'on effectue une oxydation directe, cette oxydation directe produisant la deuxième fraction de méthanol (108.2).

4. Procédé selon la revendication 1, 2 ou 3, **caractérisé en ce que** le gaz riche en oxygène (109) est stocké provisoirement.

5. Procédé selon l'une des revendications précédentes, **caractérisé en ce que** le prélèvement de dioxyde de carbone (CO₂ ; 101) s'effectue depuis un processus de combustion ou un processus d'oxydation par séparation de CO₂.

6. Procédé selon l'une des revendications 1 à 5, **caractérisé en ce que** le dioxyde de carbone (CO₂ ; 101) est prélevé depuis une installation qui libère du gaz naturel brut lors de la purification d'une fraction de dioxyde de carbone.

7. Procédé selon la revendication 1 ou 2, **caractérisé en ce que** l'électrolyse est une électrolyse de l'eau (105) au cours de laquelle on produit, directement à partir d'eau ou d'une solution aqueuse (H₂O ; 102), de l'hydrogène (H₂ ; 103) et le gaz riche en oxygène (109).

8. Procédé selon l'une des revendications précédentes, **caractérisé en ce que** l'énergie continue (E1) est fournie par la lumière du soleil au moyen d'une installation solaire thermique (400) et/ou d'une installation photovoltaïque (300).

9. Procédé selon l'une des revendications précédentes, **caractérisé en ce que** le gaz contenant des hydrocarbures est un gaz contenant du méthane.

10. Procédé selon l'une des revendications 1 à 9, **caractérisé en ce que** le gaz contenant des hydrocarbures est fabriqué à partir de matériaux contenant des carbones.

11. Procédé selon l'une des revendications 1 à 9, **caractérisé en ce que** la deuxième fraction de méthanol (108.2) est fabriquée par reformage par gaz de synthèse.

12. Procédé selon la revendication 11, **caractérisé en ce que** le reformage est un reformage autotherme.

13. Procédé selon l'une des revendications précédentes, **caractérisé en ce que**, plus tard, la première fraction de méthanol (108.1) et/ou la deuxième fraction de méthanol (108.2) se transforme en un gaz de craquage composé pour l'essentiel de monoxyde de carbone et d'hydrogène.

14. Procédé selon l'une des revendications précédentes, **caractérisé en ce que**, dans une étape intermédiaire avant la transformation en la deuxième fraction de méthanol (108.2), il est produit un gaz de synthèse qui se compose pour l'essentiel de monoxyde de carbone CO et d'hydrogène H₂, ce gaz de synthèse étant produit par une oxydation partielle des hydrocarbures.

15. Installation (700) pour produire une source énergétique stockable et transportable (108.1, 108.2), comprenant
- une installation (300, 301 ; 400) pour produire une première part énergétique sous la forme d'une énergie électrique continue (E1) à partir d'au moins une source d'énergie renouvelable,
- un dispositif d'électrolyse (102, 105) pour produire de l'hydrogène (103) comme produit intermédiaire et pour produire un gaz riche en oxygène (109), les besoins énergétiques de ce dispositif d'électrolyse (102, 105) étant pour l'essentiel, c'est-à-dire à plus de 80 %, couverts par la première part énergétique (E1),
- une alimentation en dioxyde de carbone pour amener le dioxyde de carbone (101) ;
- une zone de réaction (106) pour fabriquer une première fraction de méthanol (108.1), l'hydrogène (103) entrant en réaction avec le dioxyde de carbone (101) dans la zone de réaction (106),
- une installation de réaction (118) pour fabriquer une deuxième fraction de méthanol (108.2), le gaz riche en oxygène (109) entrant en réaction d'oxydation avec le gaz contenant des hydrocarbures (117) dans l'installation de réaction (118),
- un prélèvement (701) pour la première fraction de méthanol (108.1), et
- un prélèvement (702) pour la deuxième fraction de méthanol (108.2).

16. Installation (700) selon la revendication 15, **caractérisée en ce qu'**elle comprend des moyens pour produire du méthanol sous forme d'un mélange de la première fraction de méthanol (108.1) et de la deuxième fraction de méthanol (108.2).

17. Installation (700) selon la revendication 15 ou 16, **caractérisée en ce qu'**elle comprend une commande (110) qui est conçue pour commander les procédés qui se déroulent dans les différentes parties de l'installation.

18. Installation (700) selon la revendication 17, **caractérisée en ce qu'**il est intégré dans la commande (110) un processus décisionnel basé sur un logiciel pour qu'il soit produit entre 15 et 40 % de première fraction de méthanol (108.1) et entre 85 et 60% de deuxième fraction de méthanol (108.2).

19. Installation (700) selon l'une des revendications 15 à 18, **caractérisée en ce que** la commande (110) permet de régler et/ou de commander, par le biais de lignes de commande ou lignes de signaux (112, 113, 114, 703, 704) des courants d'énergie et/ou des débits massiques dans l'installation (700).

20. Installation (700) selon l'une des revendications 15 à 19, **caractérisée en ce qu'**elle comprend une zone de réaction pour fabriquer un gaz de synthèse, composé pour l'essentiel de monoxyde de carbone CO et d'hydrogène H₂, ce gaz de synthèse étant produit dans la zone de réaction par une oxydation partielle des hydrocarbures.

21. Installation (700) selon l'une des revendications 15 à 20, **caractérisée en ce que** l'installation de réaction (118) est conçue comme une installation pour fabriquer la deuxième fraction de méthanol (108.2) avec le gaz de synthèse par des réactions de reformage.

22. Installation (700) selon la revendication 21, **caractérisée en ce que** le reformage est un reformage autotherme.

23. Installation (700) selon l'une des revendications 15 à 22, **caractérisée en ce qu'**elle est conçue pour produire, à partir de la première fraction de méthanol (108.1) et/ou de la deuxième fraction de méthanol (108.2), un gaz de craquage composé pour l'essentiel de monoxyde de carbone et d'hydrogène.
